# EUROPEAN PATENT APPLICATION

(11) **EP 2 962 700 A1**
(43) Date of publication of application: **06.01.2016**
(21) Application number: 14382253.4
(22) Date of filing: 02.07.2014
(51) Int. Cl.: A61L 9/01

(54) **A malodour counteracting combination**

(71) Applicant: Lucta S.A., 08170 Montornes del Valles (ES)
(72) Inventor: Medina Campos, Julián, 08170 MONTORNËS DEL VALLÉS (ES); Ibáñez, Carlos, 08170 MONTORNÉS DEL VALLÉS (ES); Solá Parera, José, 08170 MONTORNÉS DEL VALLÉS (ES)
(74) Representative: ZBM Patents - Zea, Barlocci & Markvardsen

(57) **Abstract**

The present invention comprises a composition comprising a combination of hexanal, 3,7-dimethylocta-2,6-dienal, and 2,6-dimethyl-5-heptenal wherein: hexanal is in an amount from 0.1 to 4% by weight of the total weight of the composition; 3,7-dimethylocta-2,6-dienal is in an amount from 5 to 25% by weight of the total weight of the composition; and 2,6-dimethyl-5-heptenal is in an amount from 1 to 8 % by weight of the total weight of the composition; to articles containing the composition; as well as its use for counteracting malodours and a process for counteracting malodours.

## Description

The present invention relates to the field of perfumery and more particularly to the field of malodour counteraction. Especially, it relates to malodour counteracting composition capable of neutralizing in an efficient manner, through chemical reactions malodours of a large variety of origins. The invention also relates to articles containing the composition, as well as their use for counteracting malodours.

### BACKGROUND ART

There is a particular effort in the fragrance industry for the development of novel compounds to treat and control malodours. "Malodour" or "odour" are terms used to describe undesirable or unpleasant smells. Malodours are offensive odours which can be encountered in the air, in the water and on many substrates such as fabrics, hard surfaces, skin, and hair.

Common sources of malodours can include personal or environmental origin such as for example body perspiration; urine; faeces; tobacco smoke; gasoline; cooking odours; and mould and mildew. All these odours can easily be deposited on fabric, hair, and skin. Amines, thiols, sulphides, short chain aliphatic and olefin acids, aldehydes, and esters form the largest and most unpleasant chemical groups found in the above-mentioned malodours, which humans can detect.

Several approaches to counteract malodours have been disclosed. The majority of these approaches include masking by superimposing the malodour with a pleasant stronger smell; or cross-adapting by blocking of the malodour olfactory receptors; or suppressing by mixing with an ingredient that causes a negative deviation of Raoul's law; or absorption of the malodour by a porous or cage-like structure.

Although all of the approaches set forth above mitigate malodours, none of them adequately eliminates them.

Other attempts to mitigate malodours are based on the use of compounds that could have the capacity of eliminating the malodour by chemical reaction. Unfortunately, many of the compounds disclosed are not capable of neutralizing all types of functional groups contained in the common malodour molecules, and then, they are unable of counteracting malodours of large variety of origins. Additionally, many of these compounds themselves have very unpleasant and offensive odours that compromise their use in articles of common use such as household products, and/or cosmetics and personal care products.

Therefore, from what is known in the art, it is derived that there is still the need of providing malodour counteracting compositions capable of neutralizing in an efficient manner, through chemical reactions, malodours of a large variety of origins without compromising the sensorial properties for being used in articles for consume.

### SUMMARY OF THE INVENTION

Inventors have found that a composition comprising a combination of hexanal, 3,7-dimethylocta-2,6-dienal, and 2,6-dimethyl-5-heptenal wherein hexanal is in an amount from 0.1 to 4% by weight of the total weight of the composition; 3,7-dimethylocta-2,6-dienal is in an amount from 5 to 25% by weight of the total weight of the composition; and 2,6-dimethyl-5-heptenal is in an amount from 1 to 8 % by weight of the total weight of the composition allows effective neutralization of a large variety of malodours without having an unpleasant olour. The composition of the invention counteracts malodours by reaction of these compounds with the compounds responsible of the malodour in gaseous and liquid phase. It is also advantageous because the composition of the invention can be incorporated in malodour counteracting articles and being applied in spaces and on surfaces to be deodorized or freshened.

Thus, the first aspect of the present invention relates to a composition comprising a combination of hexanal, 3,7-dimethylocta-2,6-dienal, and 2,6-dimethyl-5-heptenal wherein hexanal is in an amount from 0.1 to 4% by weight of the total weight of the composition; 3,7-dimethylocta-2,6-dienal is in an amount from 5 to 25% by weight of the total weight of the composition; and 2,6-dimethyl-5-heptenal is in an amount from 1 to 8 % by weight of the total weight of the composition.

The second aspect of the present invention relates to an article comprising the composition as defined in the first aspect of the invention.

The third aspect of the invention relates to the use of the composition as defined in the first aspect of the invention; or the article as defined in the second aspect of the invention for counteracting malodours.

Finally, the fourth aspect of the invention relates to a process for counteracting malodours comprising applying an appropriate amount of the composition as defined in the first aspect of the invention to a space or surface for reducing, eliminating, or preventing malodours.

### BRIEF DESCRIPTION OF THE DRAWINGS

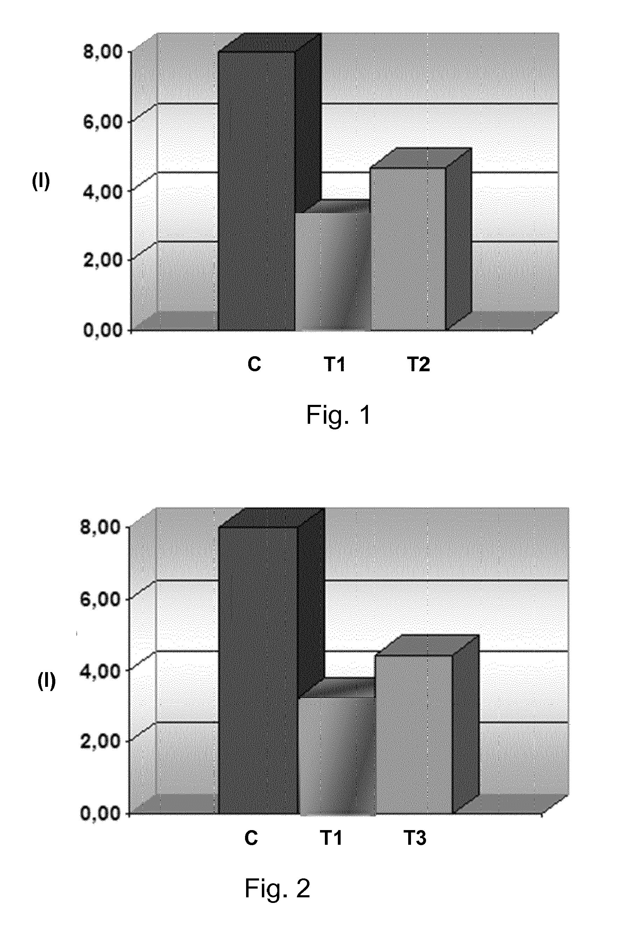

FIG. 1 shows the perceived tobacco malodour intensity in the sensorial test 1. The scheme units are shown below: "I" is tobacco odour intensity (cm); "C" represents the control booth comprising tobacco odour sample; T1 represents the test booth comprising tobacco odour sample and air freshener 1; T2 represents the test booth comprising tobacco odour sample and air freshener 2.
FIG. 2 shows the perceived tobacco malodour intensity in the sensorial test 2. The scheme units are shown below: "I" is tobacco odour intensity (cm); "C" represents the control booth comprising tobacco odour sample; T1 represents the test booth comprising tobacco odour sample and air freshener 1; T3 represents the test booth comprising tobacco odour sample and air freshener 3.

### DETAILED DESCRIPTION OF THE INVENTION

All terms as used herein in this application, unless otherwise stated, shall be understood in their ordinary meaning as known in the art. Other more specific definition terms as used in the present application are as set forth below and are intended to apply uniformly throughout the specification and claims unless an otherwise expressly set out definition provides a broader definition.

The term "weight ratio" refers to the relation in weight of a given compound to another given compound, for instance, between the compounds hexanal, 3,7-dimethylocta-2,6-dienal, and 2,6-dimethyl-5-heptenal needed to counteracting malodours.

The term "percentage (%) by weight of the total weight of the composition" refers to the percentage of each compound in relation to the total weight of the composition.

The term "percentage (%) by weight of the total weight of the combination" refers to the percentage of each compound of the combination in relation to the total weight of the combination, that is the sum of the amounts of hexanal, 3,7-dimethylocta-2,6-dienal, and 2,6-dimethyl-5-heptenal. For instant, an amount of hexanal of 4% by weight of the total weight of the composition corresponds to an amount of hexanal of 10.8% by weight of the total weight of the combination. Therefore, a composition comprising hexanal in an amount from 0.1 to 4% by weight of the total weight of the composition; 3,7-dimethylocta-2,6-dienal in an amount from 5 to 25% by weight of the total weight of the composition; and 2,6-dimethyl-5-heptenal in an amount from 1 to 8 % by weight of the total weight of the composition; corresponds to a composition comprising hexanal in an amount from 0.3 to 10.8% by weight of the total weight of the combination; 3,7-dimethylocta-2,6-dienal is in an amount from 13.5 to 67.6% by weight of the total weight of the combination; and 2,6-dimethyl-5-heptenal is in an amount from 2.7 to 21.6 % by weight of the total weight of the combination.

The term "appropriate acceptable" refers to that excipients or carriers suitable for use in perfumery, particularly in the malodour counteraction for preparing compositions with perfumery use.

The term "article" refers to any product that is intended for consumers or that can reasonably be expected to be used by consumers. Article can include air fresheners, household cleaners, detergents, softeners, soaps, bleaches, candles, toilet paper, wipes, insecticides and hygiene products for application on human skin or hair or on animal fur and skin, litter containers and animal cages.

In the context of the invention, the terms "hexanal", "caproaldehyde" and "aldehyde C-06" have the same meaning and are used interchangeable. In particular, the "hexanal" has the molecular formula C₆H₁₂O (CAS number 66-25-1), having the following structure:

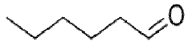

The terms "citral", "lemonal", "geranial", and "neral" have the same meaning and are used interchangeable. In particular, the term "citral" is the INN (International Nonproprietary Name) of 3,7-dimethylocta-2,6-dienal (CAS number 5392-40-5) having the following structure:

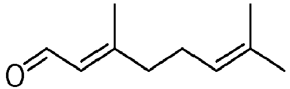

The term "melonal" is the INN (International Nonproprietary Name) of 2,6-dimethyl-5-heptenal (CAS number 106-72-9) having the following formula:

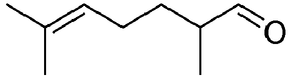

As it is illustrated in the Examples the specific combination of hexanal, citral, and melonal allows counteracting a large variety of malodours maintaining effective and pleasant sensorial counteracting malodour properties.

In an embodiment, the composition of the invention is that wherein the amount of hexanal is from 0.3 to 2% by weight of the total weight of the composition; the amount of 3,7-dimethylocta-2,6-dienal is from 15 to 20% by weight of the total weight of the composition; and the amount of 2,6-dimethyl-5-heptenal is from 3 to 6% by weight of the total weight of the composition.

In an embodiment, the composition of the invention is that wherein the amount of hexanal is 1.0% by weight of the total weight of the composition; the amount of 3,7-dimethylocta-2,6-dienal is 17.00% by weight of the total weight of the composition; the amount of and 2,6-dimethyl-5-heptenal is 4.00% by weight of the total weight of the composition. In an alternative embodiment, the composition of the invention is that wherein the amount of hexanal is 0.5% by weight of the total weight of the composition; the amount of 3,7-dimethylocta-2,6-dienal is 17.30% by weight of the total weight of the composition; and the amount of 2,6-dimethyl-5-heptenal is 4.20% by weight of the total weight of the composition.

In an embodiment, the composition further comprising dihydromyrcenol. The term "dihydromyrcenol" is the INN (International Nonproprietary Name) of 2,6-dimethyloct-7-en-2-ol (CAS number 18479-58-8) having the following structure:

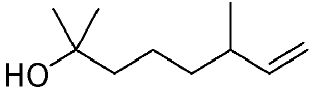

In an embodiment of the invention, the composition, which further comprises dihydromyrcenol, is that wherein the amount of dihydromyrcenol is from 10 to 40% by weight of the total weight of the composition. Preferably, the amount of dihydromyrcenol is 30% by weight of the total weight of the composition. This composition is particularly advantageous because allows neutralizing the malodours and at the same time giving a better sensorial pleasant clean scent for the use in everyday products for cleaning and personal hygiene.

In an embodiment of the invention, the composition consists of a combination of hexanal, 3,7-dimethylocta-2,6-dienal, and 2,6-dimethyl-5-heptenal wherein: hexanal is in an amount from 0.3 to 10.8% by weight of the total weight of the combination; 3,7-dimethylocta-2,6-dienal is in an amount from 13.5 to 67.6% by weight of the total weight of the combination; and 2,6-dimethyl-5-heptenal is in an amount from 2.7 to 21.6 % by weight of the total weight of the combination.

In an embodiment of the invention, the composition further comprises one or more acceptable excipients or carriers.

In an embodiment of the invention, the composition is that wherein the acceptable excipients or carriers are in an amount from 75 to 99.9% by weight of the total weight of the composition; preferably, in an amount from 80 to 95% by weight of the total weight of the composition.

The composition defined above which comprises appropriate excipients or carriers includes, but not limited to solvents, colorants, sunscreen filters, thickeners, surfactants, and preservative. The excipients or carriers used in the composition of the invention do not significantly modify the counteracting properties of the combination of the invention.

The compositions mentioned above can include one or more solvents. Examples of solvents include, but are not limited to alcohols such as ethanol, isopropanol, 3-methoxy-3-methyl-1-butanol; glycols such as butylene glycol, dipropylene glycol, dipropylene glycol methyl ether, and tripropylene glycol methyl ether; paraffin and isoparaffin waxes; silicone such as disiloxane and tetrasiloxane; isopropyl myristate; (C₁₂-C₁₅) alkyl benzoate; and mixture thereof. Preferably, the solvent is 3-methoxy-3-methyl-1-butanol,dipropylene glycol, tripropylene glycol methyl ether, isoparaffin waxes, and dipropylene glycol methyl ether.

The compositions of the invention can include one or more colorants including liposoluble and hydrosoluble colorants. Examples of colorants include, but are not limited to, fat orange (CI 12055), fat blue (CI 61554), Red Violet (CI 62025), fat yellow (CI 47000), fat green (CI 61565), and fat red (CI 26105).

The compositions of the invention can include one or more sunscreen filters. Examples of sunscreen filters include, but are not limited to, benzophenone-2, and benzophenone-3.

The compositions of the invention can include one or more thickeners. Examples of thickeners suitable for the present invention include, but are not limited to, silicon dioxide and ethyl cellulose.

The compositions mentioned above can include surfactants. Examples of surfactants suitable for the present invention include, but are not limited to, fatty alcohol ethoxylate, and derivates such as C₉-C₁₁ fatty alcohol ethoxylate (8 MOE); ethoxilated hydrogenated castor oil (40-60 MOE); and amine oxides such as mixtures of laurix/myristic amine oxide.

The compositions mentioned above can include preservatives such as antioxidants. Examples of antioxidants suitable for the present invention include, but are not limited to, butylated hydroxytoluene (BHT), butylated hydroxyanisole (BHA), and tetradibutyl pentaerithrityl hydroxyhydrocinnamate (Tinogard TT). Preferably, the preservative is butylated hydroxytoluene (BHT), and tetradibutyl pentaerithrityl hydroxyhydrocinnamate (Tinogard TT).

The compositions of the invention can be in form of a liquid, a semi-solid, or solid form. Examples include a solution, a suspension, a gel, oil, a mousse, an emulsion, and a powder.

The compositions of the present invention can be prepared according to methods well known in the state of the art. The appropriate excipients and/or carriers, and their amounts, can readily be determined by those skilled in the art according to the type of composition being prepared.

The second aspect of the invention relates to an article, which comprises the composition of the first aspect of the invention as mentioned above. The composition of the invention can be advantageously used in all the fields of modern perfumery to positively impart or modify the odour of an article into which they are incorporated.

Non-limiting examples of articles of the present invention include air fresheners, cleaners, detergents, softeners, soaps, bleaches, candles, toilet paper, wipes, insecticides, and hygiene products for application on human skin or hair or on animal fur and skin, litter containers and animal cages. Examples of air fresheners suitable for the present invention include, but are not limited to, gel air fresheners, aerosol air fresheners, electric air fresheners, membrane air fresheners, and wick air fresheners. Examples of cleaners suitable for the present invention include, but are not limited to, surface cleaners such as kitchen cleaning, and bathroom cleaning. Examples of detergents such as liquid detergents, powder detergents, compact detergents, hand and machine wash detergents, and dishwasher machine. Examples of soaps suitable for the present invention include, but are not limited to, laundry soaps, and cosmetic soaps. Examples of wipes suitable for the present invention include, but are not limited to, personal care wipes, and household cleaner wipes. Examples of insecticides suitable for the present invention include, but are not limited to, aerosol insecticides, electric insecticides, membrane insecticides, and cellulosic insecticides. Examples of hygiene products suitable for the present invention include, but are not limited to, deodorants, anti-perspirants, and shampoos.

The present invention also relates to the use of the composition as defined above, or the article of the present invention, for counteracting malodours. Particularly, the malodour counteraction is carried out by reducing, eliminating or preventing malodours. As it is shown in the Examples the composition and articles of the invention allows neutralizing malodours such as dimethylsulphide, nicotine (tobacco) and trimethylamine.

The present invention also relates to a process for counteracting malodours. Said process comprises applying an appropriate amount of the composition of the invention to a space or surface for reducing, eliminating, or preventing malodours.

In an embodiment of the invention, the process as defined above comprises applying the composition of the present invention to a surface, being the surface an inert or body surface. Examples of inert surface can be selected from kitchen or toilet surfaces, rubbish containers surfaces, textile and laundry surfaces, glass windows, dishes, and crockery surfaces.

The term "body surface" refers to any surface of the human or animal body, which may serve as a substrate for applying the composition of the invention. Examples of body surface include human skin or hair, and animal skin or fur.

In an embodiment of the invention, the process as defined above comprises applying the composition of the present invention to a space, being the space a closed space such as for example rooms and cupboards.

The composition as defined above can be applied directly to the surface to deodour; or alternatively they can be diluted in water in a previous step before their application.

Throughout the description and claims the word "comprise" and variations of the word, are not intended to exclude other technical features, additives, components, or steps. Furthermore, the word "comprise" encompasses the case of "consisting of". Additional objects, advantages, and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### 1. Counteracting Malodour Compositions

### 1A. Compositions of the invention 1-2

Table 1 shows the qualitative and quantitative composition of the counteracting malodour compositions 1-2 of the present invention

**Table 1**

| **INGREDIENTS** | | **COMPOSITION (g)** | |
|---|---|---|---|
| **NAME** | **CAS** | **1** | **2** |
| **hexanal** | 66-25-1 | **1.00** | **0.50** |
| **Citral⁽¹⁾** | 5392-40-5 | **17.00** | **17.30** |
| **Melonal⁽²⁾** | 106-72-9 | **4.00** | **4.20** |
| **dihydromyrcenol** | 18479-58-8 | 30.00 | 30.00 |
| Linalool | 78-70-6 | 34.00 | 34.00 |
| omega-penta decalactone⁽³⁾ | 106-02-5 | 10.00 | 10.00 |
| Armoise⁽⁴⁾ | 84775-75-7 | 4.00 | 4.00 |
| I-menthol⁽⁵⁾ | 2216-51-5 | 10.00 | 10.00 |
| hexenyl-3-cis acetate | 1708-82-3 | - | - |
| **heptanal** | 111-71-7 | - | - |
| **Hexyl cinnamaldehyde** | 101-86-0 | - | - |
| ethyl caprylate | 106-32-1 | - | - |
| Geraniol extra | 9007-92-5 | - | - |
| Linalool acetate | 115-95-7 | - | - |
| Orange distillate ac. Es. | | - | - |
| Nerol pure | 106-25-2 | - | - |
| Terpineol supreme | 98-55-5 | - | - |
| Total weight (gr) | | 110 | 110 |

| | | | |
|---|---|---|---|
| ⁽¹⁾ 2,6-dimethyl-5-heptenal; ⁽²⁾ 3,7-dimethylocta-2,6-dienal ;⁽³⁾exaltolide pure 941962; ⁽⁴⁾ artemisia herba-alba oil; ⁽⁵⁾ menthol 42/44 natural. | | | |

### 1 B. Comparative compositions 3-5

Table 2 shows the qualitative and quantitative composition comparative counteracting malodour compositions 3-5.

**Table 2**

| **INGREDIENTS** | | **COMPOSITION (g)** | | |
|---|---|---|---|---|
| **NAME** | **CAS** | **3** | **4** | **5** |
| **hexanal** | 66-25-1 | - | **-** | **-** |
| **Citral⁽¹⁾** | 5392-40-5 | - | **10.00** | **5.00** |
| **Melonal⁽²⁾** | 106-72-9 | - | **2.00** | **-** |
| **d ihydromyrcenol** | 18479-58-8 | 20.00 | 30.00 | 9.40 |
| Linalool | 78-70-6 | 46.00 | 34.00 | 30.00 |
| omega-penta decalactone⁽³⁾ | 106-02-5 | 10.00 | 10.00 | - |
| Armoise⁽⁴⁾ | 84775-75-7 | 4.00 | 4.00 | - |
| I-menthol⁽⁵⁾ | 2216-51-5 | 20.00 | 10.00 | - |
| hexenyl-3-cis acetate | 1708-82-3 | - | - | 0.60 |
| **heptanal** | 111-71-7 | - | - | 2.00 |
| **Hexyl cinnamaldehyde** | 101-86-0 | - | - | 1.00 |
| ethyl caprylate | 106-32-1 | - | - | 7.50 |
| Geraniol extra | 9007-92-5 | - | - | 5.00 |
| Linalool acetate | 115-95-7 | - | - | 15.00 |
| Orange distillate ac. Es. | | - | - | 4.50 |
| Nerol pure | 106-25-2 | - | - | 15.00 |
| Terpineol supreme | 98-55-5 | - | - | 5.00 |
| Total weight (gr) | | 100 | 100 | 100 |

| | | | | |
|---|---|---|---|---|
| ⁽¹⁾ 2,6-dimethyl-5-heptenal; ⁽²⁾ 3,7-dimethylocta-2,6-dienal ;⁽³⁾exaltolide pure 941962; ⁽⁴⁾ Artemisia herbal-alba oil; ⁽⁵⁾ menthol 42/44 natural. | | | | |

### 1C. Composition of the component Armoise

The component "Armoise" of the compositions 1-5 as mentioned in sections 1A, and 1 B is the INN name of the Artemisia herbal-alba oil (CAS number 84775-75-7). It comprises the following components:

| **COMPONENT** | **CAS NUMBER** | **CONTENT (%)** |
|---|---|---|
| Thujone (alpha+beta) | 546-80-5 | 55,00 |
| Camphor | 76-22-2 | 33,20 |
| Camphene | 79-92-5 | 6,00 |
| Eucalyptol | 470-82-6 | 4,10 |
| p-Cymene | 99-87-6 | 0,60 |
| 4-Terpinenol | 562-74-3 | 0,50 |
| alpha-Terpinene | 99-86-5 | 0,20 |
| gamma-Terpinene | 99-85-4 | 0,20 |
| alpha-Pinene | 80-56-8 | 0,10 |
| beta-Pinene | 127-91-3 | 0,10 |
| Total weight | | 100.00 |

### 1 D. Preparation Process

Compositions of the present invention 1-2 and comparative compositions 3-5 disclosed in sections 1A and 1 B were prepared by subsequently addition of the ingredients of corresponding compositions at room temperature.

### 2. Counteracting Malodour Air freshener

### 2A. Air freshener composition

Table 3 shows the qualitative and quantitative composition of an article containing a composition of the invention, for instant air freshener 1, and the comparative air freshener 2-3. The amount of the components is expressed in percentage by weight.

**Table 3**

| **Components** | | **Air freshener** | | |
|---|---|---|---|---|
| | | **Example** | **Comparative example** | |
| | | **1** | **2** | **3** |
| **Ethanol** | | 10.00% | 10.00% | 10.00% |
| **Counteracting malodour composition** | **2** | 0.05% | - | - |
| | **4** | - | - | 0.05% |
| | **5** | - | 0.05% | - |
| **Butane/propane** | | 89.95% | 89.95% | 89.95% |
| Total weight (ml) | | 100 | 100 | 100 |

### 2B. Preparation Process

Air fresheners 1-3 were prepared following the process as disclosed below:
(a) mixing ethanol with the counteracting malodour composition at room temperature;
(b) filling the aerosol container with the mixture obtained in step (a); and
(c) introducing the mixture of butane/propane gas in the aerosol container obtained in step (b).

### 3. Malodour Chemical Suppression Test

### A. Material

Dimethyl sulphide and nicotine (as a tobacco tracer) were used as ambient malodour generating molecules.

Compositions 1-2, and comparative compositions 3-4 were tested as malodour counteracting compositions.

### B. Method

### B1. DIMETHYL SULPHIDE SUPPRESSION TEST

### CONTROL TEST

1 microliter of pure dimethyl sulphide (0.85mg) was deposited in a 1.5 cm x 5 cm cellulose plate hanged into a 20 l desiccator chamber with a plastic covered iron wire. In the bottom of the desiccator chamber, a fan was used to remove and mix the air during the complete process.

Dimethyl sulphide was left evaporating during 5 min. After this time, the cellulose plate was taken out through a hole on the upper part of the desiccator chamber, and the hole was closed with a plug. After 15 min, a measure of the dimethyl sulphide level in the chamber ambient was taken using solid phase micro-extraction, SPME, with a Carboxen/DVB/PDMS fibre. The fibre was left extracting the chamber air for 15 min.

To analyse the volatiles extracted, the fibre was injected into the injection port of a gas chromatograph equipped with a FID detector. A Zebron ZB Wax Plus, 30 m x 0.25 mm x 0.25 microns chromatographic column was used. The split was 1/15 and a 1 mm i.d. glass insert was employed. The fibre was desorbed 10 min at 250°C and the oven temperature was maintained isothermally at 60°C. Dimethyl sulphide appears at around 2 min. In this way, a measure of the bad odour was obtained without using the suppressing accord. Finally, the desiccator chamber was opened and cleaned in a fume hood.

### SAMPLE TEST

1 microliter of pure dimethyl sulphide (0.85mg) was deposited over a 1.5 cm x 5 cm cellulose plate and hanged into the 20 I desiccator chamber with a plastic covered iron wire. In the bottom of the desiccator chamber, a fan was also used to remove and mix the air. Dimethyl sulphide was left evaporating during 5 min. After this time, the cellulose plate was taken out through the hole on the upper part of the desiccator chamber, and another 3.0 cm x 5 cm cellulose plate was introduced through the same hole, containing 1 g of the composition to test. The plate was hanged with the same wire and the hole was closed with the plug.

After 15 min, the second cellulose plate was taken out through the hole on the upper part of the desiccator chamber and a measure of the dimethyl sulphide level in the chamber ambient was taken using solid phase micro-extraction, SPME. The fibre was left extracting the chamber air for 15 min. The fibre was desorbed 10 min at 250°C into the GC/FID and volatiles were analysed.

If it were a suppression of the malodour by the tested composition, the dimethyl sulphide level would decrease in the chamber air comparing with the control test.

### B2. NICOTINE SUPPRESSION TEST

10 microliters of pure nicotine was deposited over a 1.5 cm x 5 cm cellulose plate and hanged into the 20 l desiccator chamber with a plastic covered iron wire. In the bottom of the desiccator chamber, a fan was also used to remove and mix the air. Nicotine was left evaporating during 30 min. After this time, the cellulose plate was taken out through the hole on the upper part of the desiccator chamber, and another 3.0 cm x 5 cm cellulose plate was introduced through the same hole, containing 1 g of the composition to test.

The plate was hanged with the same wire and the hole was closed with the plug. After 30 min, the second cellulose plate was taken out through the hole on the upper part of the desiccator chamber and a measure of the nicotine level in the chamber ambient was taken using solid phase micro-extraction, SPME. The fibre was left extracting the chamber air for 30 min. The fibre was desorbed 10 min at 250°C into the GC/FID and volatiles were analysed with an oven program from 60°C to 230°C.

The same process was repeated without composition to test for comparing results (CONTROL TEST).

### B3. TRIMETHYLAMINE SUPPRESSION TEST

2 microliters of trimethylamine 45% in water was deposited over a 1.5 cm x 5 cm cellulose plate and hanged into the 20 I desiccator chamber with a plastic covered iron wire. In the bottom of the desiccator chamber, a fan was also used to remove and mix the air. Trimethylamine was left evaporating during 5 min. After this time, the cellulose plate was taken out through the hole on the upper part of the desiccator chamber, and another 3.0 cm x 5 cm cellulose plate was introduced through the same hole, containing 1 g of the fragrance accord to test. The plate was hanged with the same wire and the hole was closed with the plug.

After 30 min, the second cellulose plate was taken out through the hole on the upper part of the desiccator chamber and a measure of the trimethylamine level in the chamber ambient was taken using solid phase micro-extraction, SPME. The fibre was left extracting the chamber air for 10 min. The fibre was desorbed 10 min at 250°C into the GC/FID and volatiles were analysed isothermally at 60°C.

The same process was repeated without composition to test for comparing results (CONTROL TEST).

### C. Results

### C1. DIMETHYL SULPHIDE SUPPRESSION

The decrease of the amount of dimethyl sulphide in the chamber indicates the percentage of suppression of the malodour (dimethyl sulphide) by the tested composition compared with the control test. It is observed by the decrease of the final area at 15 min.

Table 4 shows the percentage of suppression of the dimethyl sulphide by the tested compositions.

**Table 4**

| **Composition** | Dimethyl sulphide in the cellulosic plate (g) | final Area 15 min | Suppression (%) |
|---|---|---|---|
| Control | 0g | 2.22 | 0.0 |
| **Composition 1** | 0.996 | 0.00 | **100** |
| **Composition 2** | 1.027 | 0.46 | **79.3** |
| Comparative Composition 3 | 1.003 | >2.22 | 0.0 |
| Comparative composition 4 | 1.016 | 2.38 | 0.0 |

The results of the Table 4 show that only the compositions of the invention 1-2 which comprises the specific combination of the aldehydes hexanal, citral, and melonal within the claimed ranges allows suppressing dimethyl sulphide. In contrast, comparative compositions, including those that only comprise two of the three claimed aldehydes (comparative composition 4), do not allow dimethyl sulphide suppression as it is observed by the no reduction of the final area.

### C2. NICOTINE SUPPRESSION

The decrease of the amount of nicotine in the chamber indicates the percentage of suppression of the malodour (nicotine) by the tested composition compared with the control test.

Table 5 shows the percentage of suppression of the nicotine by the tested compositions.

**Table 5**

| **Composition** | nicotine in the cellulosic plate (g) | final Area 30 min | Suppression (%) |
|---|---|---|---|
| Control | 0 | 1904.4 | 0.0 |
| **Composition 2** | 1.018 | 1414.5 | **25.7** |
| Comparative Composition 3 | 1.015 | 2040.4 | 0.0 |
| Comparative composition 4 | 1.013 | 1971.5 | 0.0 |
| Comparative composition 5 | 1.016 | 1881.9 | 1.2 |

The results of the Table 5 show that only the compositions of the invention that comprise the specific combination of the aldehydes hexanal, citral, and melonal within the claimed ranges allows suppressing nicotine. In contrast, comparative compositions, including those that only comprises two of the three claimed aldehydes (comparative composition 4); or comparative composition which only comprises one of the claimed aldehydes and two aldehydes known as malodour counteracting agents (comparative 5) do not allow nicotine suppression.

### C3. TRIMETHYLAMINE SUPPRESSION

The decrease of the amount of trimethylamine in the chamber indicates the percentage of suppression of the malodour (trimethylamine) by the tested composition compared with the control test.

Table 6 shows the percentage of suppression of the trimethylamine by the tested compositions.

**Table 6**

| **Composition** | trimethylamine in the cellulosic plate (g) | final Area 30 min | Suppression (%) |
|---|---|---|---|
| Control | 0 | 4.20 | 0.0 |
| **Composition 2** | 1.026 | 1.88 | **55.2** |
| **Composition 3** | 1.076 | 1.95 | **53.6** |
| Comparative Composition 3 | 1.015 | 2.53 | 37.7 |

The results of the Table 6 show that only the compositions of the invention that comprises the specific combination of the aldehydes hexanal, citral, and melonal within the claimed ranges allows efficient suppression of trimethylamine.

### D. CONCLUSION OF SUPPRESSION TEST

Compositions of the invention comprising the combination of the aldehydes hexanal, citral, and melonal within the claimed ranges allows effective suppression of a large variety of malodours regardless of their origin.

### 3. Malodour sensorial Suppression Test

### A. Material

Tobacco odour sample: 6 g of blond and black cigarette butts litter plus 1 g of tobacco ashes.

Air freshener samples: Air freshener 1, 2, and 3.

### B. Method

The sensorial test was performed in three equal booths of 3.78 x 2.75 x 2.60 = 27 m³. Tobacco odour sample was sprayed in each booth and was left into the booths for 30 minutes. After this time, each air freshener 1-3 was separately sprayed in each booth. The total air freshener amount dosed is controlled by weight. The content of each booth is defined as follows:

### TEST 1:

- CONTROL BOOTH:
   - Tobacco odour sample (CONTROL BOOTH)
- TEST BOOTHS:
   - Tobacco odour sample and air freshener 1 (T1); sprayed Dose:1.95g;
   - Tobacco odour sample and air freshener 2 (T2); sprayed dose:1.87g.

TEST 2:
- CONTROL BOOTH:
   - Tobacco odour sample (CONTROL BOOTH)
- TEST BOOTHS:
   - Tobacco odour sample and air freshener 1 (T1); sprayed dose:1.55g;
   - Tobacco odour sample and air freshener 3 (T3); sprayed dose: 1.51 g.

Each panellist is asked to score the tobacco odour level on a 0-10 cm continuous scale whose edges would correspond to the following odour levels: 0 cm = No tobacco odour; and 10 cm = Very strong malodour.

### C. Results

The tobacco odour intensity is tested in each booth.

The intensity results of test 1 are summarized in Table 7 and in Fig.1.

**Table 7**

| **Panellist** | **BOOTH** | | |
|---|---|---|---|
| | **Control** | **T1** | **T2** |
| 1 | 8.9 | 1.4 | 2.2 |
| 2 | 9.3 | 5.2 | 6.5 |
| 3 | 9.3 | 4.5 | 5.5 |
| 4 | 8.5 | 5.4 | 7.0 |
| 5 | 8.4 | 4.7 | 5.2 |
| 6 | 6.5 | 1.4 | 2.7 |
| 7 | 8.4 | 2.8 | 4.3 |
| 8 | 9.3 | 2.4 | 4.8 |
| 9 | 8.6 | 1.7 | 3.5 |
| 10 | 7.5 | 1.8 | 3.6 |
| 11 | 8.7 | 4.2 | 6.2 |
| 12 | 8.6 | 5.2 | 3.2 |
| 13 | 6.2 | 3.2 | 5.4 |
| 14 | 8.4 | 1.0 | 2.1 |
| 15 | 8.2 | 3.2 | 7.1 |
| 16 | 8.4 | 3.2 | 6.1 |
| **Means scores** | **8.32** | **3.20** | **4.71** |
| **% Reduction** | | **61.56%** | **43.42%** |

The intensity results of test 2 are summarized in Table 8 and in Fig. 2.

**Table 8**

| | **BOOTH** | | |
|---|---|---|---|
| **Panellist** | **Control** | **T1** | **T3** |
| 1 | 6.8 | 2.7 | 3.7 |
| 2 | 9.8 | 4.1 | 7.2 |
| 3 | 6.8 | 1.8 | 2.5 |
| 4 | 5.8 | 0.8 | 3.8 |
| 5 | 7.8 | 3.5 | 3.1 |
| 6 | 9.8 | 5.9 | 5.2 |
| 7 | 6.8 | 2.8 | 3.5 |
| 8 | 8.0 | 4.0 | 5.2 |
| 9 | 8.5 | 1.8 | 3.4 |
| 10 | 8.1 | 2.1 | 1.2 |
| 11 | 9.2 | 6.7 | 7.0 |
| 12 | 8.4 | 3.2 | 6.7 |
| 13 | 9.6 | 5.2 | 8.2 |
| 14 | 8.6 | 1.8 | 3.2 |
| 15 | 6.7 | 2.1 | 2.9 |
| **Means scores** | **8.04** | **3.23** | **4.45** |
| **% Reduction** | | **59.80%** | **44.65%** |

The results of Table 7 and 8 show that the article (air fresheners) of the present invention, which comprises the combination of hexanal, 3,7-dimethylocta-2,6-dienal, and 2,6-dimethyl-5-heptenal within the claimed ranges reduces significantly the perceived malodour intensity of tobacco even more than the comparative air freshener compositions 4 and 5, which do not comprises the combination of the invention.

## Claims

1. A composition comprising a combination of hexanal, 3,7-dimethylocta-2,6-dienal, and 2,6-dimethyl-5-heptenal wherein:
hexanal is in an amount from 0.1 to 4% by weight of the total weight of the composition;
3,7-dimethylocta-2,6-dienal is in an amount from 5 to 25% by weight of the total weight of the composition; and
2,6-dimethyl-5-heptenal is in an amount from 1 to 8 % by weight of the total weight of the composition.

2. The composition according to claim 1, wherein:
hexanal is in an amount from 0.3 to 2% by weight of the total weight of the composition;
3,7-dimethylocta-2,6-dienal is in an amount from 15 to 20% by weight of the total weight of the composition; and
2,6-dimethyl-5-heptenal is in an amount from 3 to 6% by weight of the total weight of the composition.

3. The composition according to any of the claims 1-2, wherein:
hexanal is in an amount of 0.5% by weight of the total weight of the composition;
3,7-dimethylocta-2,6-dienal is in an amount of 17.30% by weight of the total weight of the composition; and
2,6-dimethyl-5-heptenal is in an amount of 4.20% by weight of the total weight of the composition.

4. The composition according to any of the claims 1-3, further comprising 2,6-dimethyloct-7-en-2-ol.

5. The composition according to claim 4, wherein 2,6-dimethyloct-7-en-2-ol is in an amount from 10 to 40% by weight of the total weight of the composition.

6. The composition according to claim 5, wherein 2,6-dimethyloct-7-en-2-ol is in an amount of 30% by weight of the total weight of the composition.

7. A composition according to claim 1, consisting of a combination of hexanal, 3,7-dimethylocta-2,6-dienal, and 2,6-dimethyl-5-heptenal wherein:
hexanal is in an amount from 0.3 to 10.8% by weight of the total weight of the combination;
3,7-dimethylocta-2,6-dienal is in an amount from 13.5 to 67.6% by weight of the total weight of the combination; and
2,6-dimethyl-5-heptenal is in an amount from 2.7 to 21.6 % by weight of the total weight of the combination.

8. The composition as defined in any of the claims 1-6, further comprising one or more acceptable excipients or carriers.

9. The composition according to claim 8, wherein the acceptable excipients or carriers are in an amount from 75 to 99.9% by weight of the total weight of the composition.

10. The composition according to any of the claims 8-9, wherein the acceptable excipients or carriers are in an amount from 80 to 95% by weight of the weight of the composition.

11. The composition according to any of the claims 8-10, wherein the excipients or carriers are selected from the group consisting of solvents, colorants, sunscreen filters, thickeners, surfactants, and preservative, and mixture thereof.

12. An article comprising the composition of any of the claims 1-11, which is in form of air fresheners, household cleaners, detergents, softeners, soaps, bleaches, candles, toilet paper, wipes, insecticides, and hygiene products for application on human skin or hair or on animal fur and skin, litter containers and animal cages.

13. Use of the composition as defined in any of the claims 1-11, or alternatively the article as defined in claims 12 for counteracting malodours.

14. Use according to claim 13, wherein the counteracting malodours is carried out by reducing, eliminating, or preventing malodours.

15. A process for counteracting malodours comprising applying an appropriate amount of the composition as defined in any of the claims 1-11, to a space or surface for reducing, eliminating, or preventing malodours.
